**Europäisches Patentamt**

**European Patent Office**

**Office européen des brevets**

(19)

(11) Veröffentlichungsnummer : **0 028 308**
**B1**

(12) **EUROPÄISCHE PATENTSCHRIFT**

(45) Veröffentlichungstag der Patentschrift :
**11.05.83**

(21) Anmeldenummer : **80105657.3**

(22) Anmeldetag : **20.09.80**

(51) Int. Cl.³ : **C 07 C 49/92,** C 07 C 45/77,
C 07 F 15/06

(54) **Verfahren zur Herstellung von Cobalt(III)-acetylacetonat.**

(30) Priorität : **19.10.79 NL 7907742**

(43) Veröffentlichungstag der Anmeldung :
**13.05.81 Patentblatt 81/19**

(45) Bekanntmachung des Hinweises auf die Patenterteilung : **11.05.83 Patentblatt 83/19**

(84) Benannte Vertragsstaaten :
**AT BE CH DE FR GB IT LI LU NL SE**

(56) Entgegenhaltungen :
**DE B 2 420 691**

(73) Patentinhaber : **Chemische Fabriek Zaltbommel B.V.**
**Postbus 52 Koxkampseweg 14**
**Zaltbommel (NL)**

(72) Erfinder : **van der Maas, Hendrikus J.**
**De Boogerd 25**
**NL-5305 CK Zuilichem Gemeinde Brakel (NL)**

(74) Vertreter : **Siecke, Wolf-Friedrich, Dr.**
**c/o DYNAMIT NOBEL AKTIENGESELLSCHAFT HA**
**Patente/Patentabteilung Postfach 1209 Kölner**
**Strasse**
**D-5210 Troisdorf/Bez. Köln (DE)**

## Verfahren zur Herstellung von Cobalt(III)-acetylacetonat

Gegenstand der vorliegenden Erfindung ist ein Verfahren zur Herstellung von Cobalt(III)-acetylacetonat, bei dem Cobalt(II)-acetylacetonat mit Wasserstoffperoxid in Gegenwart von Acetylaceton zu Cobalt(III)-acetylacetonat umgesetzt wird. Die Umsetzung findet mit gelöstem Ausgangsprodukt statt, das auf spezielle Weise hergestellt wurde.

Es ist aus der DE-AS 24 20 691 (NOA 75 03 276) bekannt, Cobalt(II)-acetylacetonat mit Wasserstoffperoxid und Acetylaceton zu Cobalt(III)-acetylacetonat umzusetzen. Dabei geht man von einem Cobalt(II)-acetylacetonat aus, das durch Umsetzung eines Cobalt(II)-salzes in wässriger Lösung mit Acetylaceton nach bekannten Verfahren hergestellt wurde. Ein auf diese Weise hergestelltes Cobalt(II)-acetylacetonat enthält immer zwei Mol Kristallwasser, die nur auf sehr aufwendige Weise entfernbar sind.

Weiterhin wird bei diesem bekannten Verfahren in beliebigen organischen Lösungsmitteln die Oxidation durchgeführt, wobei die bevorzugten Lösungsmittel aromatische Kohlenwasserstoffe oder Alkohole sind. Bei diesem Verfahren wirkt sich nachteilig aus, daß von einem vorgefertigten Cobalt(II)-acetylacetonat ausgegangen werden muß, dessen Herstellung bereits sehr aufwendig ist. Bei diesen bekannten Herstellungsverfahren muß nämlich das Reaktionsprodukt mit Ammoniak neutralisiert werden und anschließend müssen die Ammoniumsalze sorgfältig ausgewaschen werden.

Trotz des Einsatzes von reinem Cobalt(II)-acetylacetonat erhält man bei dem Verfahren der DE-AS 24 20 691 nur Ausbeuten zwischen 75 und 85 % an wasserfreiem Cobalt(III)-acetylacetonat.

Es bestand demzufolge die Aufgabe, Cobalt(III)-acetylacetonat so herzustellen, daß man Ausbeuten möglichst über 95 % erhält ; weiterhin bestand die Aufgabe, ein Verfahren zur Herstellung von Cobalt(III)-acetylacetonat zu finden, bei dem man nicht von einem isolierten Cobalt(II)-acetylacetonat ausgehen muß.

In Erfüllung dieser Aufgabe wurde nun ein Verfahren zur Herstellung von Cobalt(III)-acetylacetonat durch Oxidation eines Cobalt(II)-acetylacetonats in einem organischen Lösungsmittel mit Wasserstoffperoxid in Gegenwart von Acetylaceton gefunden, das dadurch gekennzeichnet ist, daß die Oxidation im Temperaturbereich zwischen 40 und 100 °C durchgeführt wird und daß man als organisches Lösungsmittel ein mit Wasser mischbares und gegenüber Wasserstoffperoxid bei Temperaturen bis 120 °C inertes organisches Lösungsmittel einsetzt, in dem das Cobalt(II)-acetylacetonat hergestellt wurde und in Lösung vorliegt. Bei Anwendung dieses erfindungsgemäßen Verfahrens ist es möglich, direkt aus einer Cobalt(II)-verbindung zu dem Cobalt(III)-acetylacetonat zu gelangen, ohne daß ein Zwischenprodukt isoliert zu werden braucht und ohne daß eine aufwendige Zwischenreinigungsstufe durchgeführt wird. Man erhält dabei Ausbeuten, die über 95 %, bezogen auf die Cobalt(II)-verbindung liegen. Das erhaltene Verfahrensprodukt ist praktisch wasserfrei und enthält kein Cobalt(II)-salz.

Wesentlich bei dem vorliegenden Verfahren ist die Wahl des entsprechenden Lösungsmittels. Die in der DE-AS 24 20 691 wörtlich genannten Lösungsmittel eignen sich nicht zur Durchführung des erfindungsgemäßen Verfahrens ; sie sind entweder nicht mit Wasser mischbar oder gegenüber Wasserstoffperoxid bei erhöhten Temperaturen nicht inert.

Zu den erfindungsgemäß einsetzbaren Lösungsmitteln zählen niedere Ketone wie Aceton oder Methyläthylketon. Weiterhin eignen sich Dioxan, Dioxolan oder Tetrahydrofuran. Das bevorzugte Lösungsmittel ist Aceton.

Das erfindungsgemäße Verfahren ist weiterhin dadurch gekennzeichnet, daß man von einem Cobalt(II)-acetylacetonat ausgeht, das in nicht isolierter Form eingesetzt wird. Diese Maßnahme wird dadurch erreicht, daß man die Herstellung des Cobalt(II)-acetylacetonats in dem beanspruchten Lösungsmittel durchführt. Die Herstellung selbst erfolgt in an sich bekannter Weise durch Umsetzung einer Cobalt(II)-verbindung mit Acetylaceton bei erhöhter Temperatur. Dabei wird als Cobaltverbindung $Co(OH)_2$, CoO oder basisches Cobalt(II)-carbonat eingesetzt. Die Reaktionstemperatur liegt zwischen 20 und 100 °C ; bevorzugt wird bei der Siedetemperatur des Lösungsmittel gearbeitet. Dabei kann man auch so vorgehen, daß man das verdampfende Lösungsmittel in der Weise kondensiert, daß das Kondensat über ein wasserabsorbierendes Mittel läuft, das das gleichzeitig verdampfte Wasser festhält ; das übrige Kondensat gelangt wieder in den Reaktionsbehälter. Auf diese Weise ist es möglich, den Ablauf der Reaktion zu kontrollieren und das Ende der Reaktion, die zur Bildung des Cobalt(II)-acetylacetonats führt, zu erkennen.

Die Menge des Acetylacetons, die mit der Cobalt(II)-verbindung dem Reaktionsgemisch hinzugefügt wird, ist mindestens die stöchiometrisch notwendige Menge zur Herstellung des Cobalt(II)-acetylacetonats. Es kann aber auch gleich zu Beginn der Gesamtreaktion diejenige Menge an Acetylaceton eingesetzt werden, die stöchiometrisch zur Bildung des gewünschten Cobalt(II)-acetylacetonats notwendig ist.

Bei der Arbeitsweise in dem erfindungsgemäßen Lösungsmittel bildet sich intermediär ein kristallwasserfreies Cobalt(II)-acetylacetonat. Dieses befindet sich in Lösung in dem beanspruchten Lösungsmittel und wird in dieser Lösung ohne Isolation auf an sich bekannte Weise mit $H_2O_2$ zu Cobalt(III)-acetylacetonat oxidiert. Die Oxidation wird in Gegenwart der stöchiometrisch notwendigen Menge von Acetylaceton durchgeführt.

Bei dieser Reaktion wird im Temperaturbereich zwischen 40 und 100 °C, vorzugsweise zwischen 50 und 90 °C gearbeitet. Zweckmäßigerweise wird bei der Siedetemperatur des Lösungsmittels gearbeitet.

Das Wasserstoffperoxid wird bevorzugt in 25 bis 35 %iger wässriger Lösung hinzugefügt. Es genügt bereits ein nur geringer Überschuß von bis zu 10 Gew.-%, um eine vollständige Oxidation zu erhalten. Im allgemeinen wird ein 1,1 bis 1,5 molarer Überschuß eingesetzt.

Es ist zweckmäßig, das Wasserstoffperoxid portionsweise hinzuzufügen, so daß es immer sofort nach der Zugabe abreagieren kann. Die Zugabe des Wasserstoffperoxids wird dann beendet, wenn kein Cobalt(II)-salz mehr anwesend ist. Dies kann durch die Dünnschichtchromatographie einer Probe leicht nachgeprüft werden.

Wenn das gesamte Cobalt(II)-salz zu Cobalt(III)-salz oxidiert ist, wird auf Temperaturen zwischen 25 und 10 °C abgekühlt. Dabei fällt dann das Cobalt(III)-acetylacetonat als wasserfreies Salz an. Es kann leicht bei Temperaturen bis zu 90 °C getrocknet werden. Das Trocknen wird durch Arbeiten im Vakuum erleichtert, wobei ein Vakuum bis herab zu 30 mbar vollkommen ausreicht.

Die Mutterlauge eines Ansatzes kann nach Abtrennen der Cobalt(III)-acetylacetonat-kristalle bedenkenlos für einen neuen Einsatz eingesetzt werden ; irgendwelche Nebenprodukte, die bei der Reaktion stören könnten, treten in nennenswertem Umfang nicht auf.

Beispiel 1

In einem mit einem Rückflußkühler und Destillationsbrücke verschenen Reaktionsgefäß werden 93 g Cobalt-hydroxid in 300 ml Aceton suspendiert. Unter Rühren werden 315 g Acetylaceton hinzugefügt ; dabei tritt eine leichte Erwärmung ein.

Das Gemisch wird zum Rückfluß erhitzt ; das abdestillierende Aceton wird über Calciumchlorid geleitet, um das mitgenommene Wasser zu absorbieren. Nach 2 Stunden erhitzen auf Siedetemperatur hatte das Calciumchlorid eine deutliche Menge Wasser gebunden.

Daraufhin wurde durch einen Tropftrichter im Laufe von 3 bis 3 1/2 Stunden 100 g Wasserstoffperoxid als 35 %ige wässrige Lösung hinzugefügt. Dabei wurde das Reaktionsgemisch unter Rückfluß gehalten ; die Destillationsbrücke wurde entfernt. Die Innentemperatur lag zwischen 60 und 65 °C.

Nachdem insgesamt 100 g Wasserstoffperoxid-Lösung hinzugefügt wurden, wurde von einer Probe eine Dünnschichtchromatographie genommen. Sie ergab, daß kein Cobalt(II)-salz mehr anwesend war. Daraufhin wurde das Reaktionsgemisch auf 10 °C abgekühlt. Die ausfallenden grün/schwarzen Kristalle wurden abfiltriert und mit 75 ml Aceton ausgewaschen. Das Produkt ließ sich an der Luft bis auf einen Feuchtigkeitsgehalt von maximal 0,2 % trocknen. Auf diese Weise wurden 338,7 g Cobalt(III)-acetylacetonat erhalten. (Ausbeute = 95,1 %, bezogen auf eingesetztes Cobalthydroxid). Die Analyse ergab einen Metallgehalt von 16,54 bis 16,59 %. Der theoretische Werte liegt bei 16,57 %.

Beispiel 2

In der Mutterlauge und dem Waschwasser des Beispiels 1 werden 93 g Cobalthydroxid suspendiert und anschließend 315 g Acetylaceton hinzugefügt. Das Gemisch wurde unter Rühren auf Rückflußtemperatur gebracht und das dabei abdestillierte Aceton über Calciumchlorid wie im Beispiel 1 getrocknet.

Nach einer Reaktionszeit von 2 Stunden wurde mit dem Zutropfen einer 35 %igen Wasserstoffperoxid-Lösung begonnen. Dabei stieg die Innentemperatur auf 60 bis 65 °C an. Insgesamt wurden im Laufe von 3 Stunden 95 g Wasserstoffperoxid hinzugefügt.

Nach Ablauf dieser Zeit ergab ein Dünnschichtchromatogramm einer Probe, daß kein Cobalt(II)-salz mehr anwesend war. Es wurde daraufhin wieder auf 10 °C abgekühlt, wobei große schwarz gefärbte Kristalle ausfielen. Diese wurden abfiltriert und mit 80 ml Aceton ausgewaschen.

Die ausgewaschenen Kristalle wurden anschließend im Vakum von 40 mbar während 1 Stunde bei 80 °C getrocknet. Es wurden 344 g Cobalt(III)-acetylacetonat erhalten (96,6 % Ausbeute). Der Metallgehalt lag zwischen 16,48 und 16,56 %, der Wassergehalt betrug maximal 0,1 %.

Beispiel 3

Dieses Beispiel zeigt, daß die Herstellung des Cobalt(II)-acetylacetonats auch ohne Absorbtion des dabei entstandenen Wassers durchgeführt werden kann.

Es wurden 128 g basisches Cobaltcarbonat in 300 ml Aceton suspendiert. Bei Raumtemperatur werden daraufhin 210 g Acetylaceton hinzugefügt. Dabei tritt bereits Reaktion ein, die sich durch Gasentwicklung und Farbveränderung zu erkennen gibt. Das Gemisch wird auf Rückflußtemperatur erhitzt ; daraufhin werden 105 g Acetylaceton hinzugefügt und noch weiterhin 1 Stunde unter Rückfluß gehalten.

Anschließend wird über einen Tropftrichter mit dem Zutropfen von einer 35 %igen Wasserstoffperoxid-Lösung begonnen. Innerhalb 4 1/2 Stunden werden auf diese Weise 100 g Wasserstoffperoxid hinzugefügt ; nach Ablauf dieser Zeit ergab ein Dünnschichtchromatogramm, daß kein Cobalt(II)-salz mehr anwesend war.

Nach dem Abkühlen des Reaktionsgemische auf 10 °C wurden die dabei erhaltenen Kristalle abfiltriert und mit 75 ml Aceton ausgewaschen. Der erhaltene Feststoff wurde im Vakuum von 40 mbar während 1 Stunde bei 80 °C getrocknet. Es wurden 343 g (96,4 der Theorie) erhalten. Die Analyse ergab einen Metallgehalt zwischen 16,51 und 16,58 %. Der Wassergehalt lag bei maximal 0,2 %.

## Beispiel 4

Zu der Mutterlauge und dem Waschwasser des Beispiels 3 werden nach dem Trocknen über Calciumchlorid 90 ml Aceton hinzugefügt. In dieser Lösung werden 128 g basisches Cobaltcarbonat suspendiert. Unter Rühren werden anschließend 215 g Acetylaceton hinzugefügt. Es ist sofort eine Reaktion erkennbar an der Veränderung der Farbe und der Kristallform der Kristalle. Außerdem tritt Gasentwicklung ein und das Gemisch wird zusehens schlechter rührbar.

Daraufhin wird das Gemisch auf Rückflußtemperatur erhitzt und nochmals 105 g Acetylaceton hinzugefügt. Es wird noch etwa eine halbe Stunde unter Rückfluß gekocht, wobei das abdestillierende Aceton über Calciumchlorid geleitet wird. Anschließend wird mit dem Zutropfen von einer 35 %igen Wasserstoffperoxid-Lösung begonnen.

Nach Ablauf von 4 1/2 Stunden waren 92 g Wasserstoffperoxid hinzugefügt und der Test auf Cobalt(II)-salz verlief negativ. Daraufhin wurde das Reaktionsgemisch auf 10 °C abgekühlt. Dabei wurden grün/schwarz gefärbte Kristalle erhalten, die abfiltriert und mit 75 ml Aceton ausgewaschen wurden. Anschließend wurden sie bei Raumtemperatur getrocknet.

Es wurden 346 g Cobalt(III)-acetylacetonat erhalten (97,2 % der Theorie). Die Analyse ergab einen Cobaltgehalt zwischen 16,55 und 16,56 % ; der Wassergehalt lag unterhalb von 0,2 %.

## Beispiel 5

Es werden 128 g basisches Cobaltcarbonat in 400 ml Aceton aufgerührt. Das Gemisch wird unter Rühren unter Rückfluß gebracht, wobei die Innentemperatur 66 °C betrug. Es werden 315 g Acetylaceton hinzugefügt. Daraufhin wird im Laufe von 4 Stunden Wasserstoffperoxid (35 %ige Lösung) tropfenweise hinzugefügt. Die Gesamtmenge an Wasserstoffperoxid betrug nach Ablauf dieser Zeit 100 g ; anschließend wurde noch eine halbe Stunde nachreagieren gelassen. Daraufhin wurde das Reaktionsgemisch auf 15 °C abgekühlt und die erhaltenen Kristalle abfiltriert und mit 100 ml Aceton ausgewaschen. Daraufhin wurde im Vakuum von 45 mbar bei 90 °C getrocknet.

Es wurden 336 g (94,4 %) Cobalt(III)-acetylacetonat mit einem Wassergehalt von maximal 0,2 % und einem Metallgehalt von 16,54 bis 16,59 % erhalten.

Aus der verbleibenden Mutterlauge werden noch 410 ml Aceton mit einem Wassergehalt von 11,8 % abdestilliert. Beim Eindampfen dieser Mutterlauge fällt nochmals Cobalt(III)-acetylacetonat aus. Dieses wird abfiltriert und wie oben beschrieben getrocknet. Auf diese Weise werden nochmals 11 g Cobalt(III)-acetylacetonat erhalten, so daß die Gesamtausbeute 347 g = 97,5 %, bezogen auf eingesetztes Cobaltcarbonat, betrug.

## Ansprüche

1. Verfahren zur Herstellung von Cobalt(III)-acetylacetonat durch Oxidation eines Cobalt(II)-acetylacetonats in einem organischen Lösungsmittel mit Wasserstoffperoxid in Gegenwart von Acetylaceton, dadurch gekennzeichnet, daß die Oxidation im Temperaturbereich zwischen 40 und 100 °C durchgeführt wird und als organisches Lösungsmittel ein mit Wasser mischbares und gegenüber Wasserstoffperoxid bei Temperaturen bis 120 °C inertes organisches Lösungsmittel eingesetzt wird, in dem das Cobalt(II)-acetylacetonat hergestellt wurde und in Lösung vorliegt.

2. Verfahren zur Herstellung von Cobalt(III)-acetylacetonat gemäß Anspruch 1, dadurch gekennzeichnet, daß man als Lösungsmittel Aceton einsetzt.

3. Verfahren zur Herstellung von Cobalt(III)-acetylacetonat gemäß Anspruch 1 oder 2, dadurch gekennzeichnet, daß man bei der Herstellung des Cobalt(II)-acetylacetonats mit einem solchen Überschuß von Acetylaceton arbeitet, der zur Herstellung des Cobalt(III)-acetylacetonats notwendig ist.

4. Verfahren gemäß einem der Ansprüche 1 bis 3, dadurch gekennzeichnet, daß man die Umsetzung der Cobalt(II)-verbindung mit Acetylaceton bei der Siedetemperatur des Lösungsmittels unter Rückfluß durchführt.

5. Verfahren gemäß Anspruch 4, dadurch gekennzeichnet, daß man das rückfließende Lösungsmittel über ein Absorbtionsmittel für Wasser leitet.

## Claims

1. Process for the preparation of cobalt (III)-acetylacetonate by oxidation of a cobalt (II)-acetylacetonate in an organic solvent with hydrogen peroxide in the presence of acetylacetone, characterised in that the oxidation is carried out in the temperature region between 40 and 100 °C and an organic solvent miscible with water and inert with respect to hydrogen peroxide at temperatures up to 120 °C in which the cobalt (II)-acetylacetonate was produced and is present in solution, is employed as organic solvent.

2. Process for the preparation of cobalt (III)-acetylacetonate according to claim 1, characterised in that acetone is employed as solvent.

3. Process for the preparation of cobalt(III)-acetylacetonate according to claim 1 or 2, characterised in that, in the preparation of the cobalt(II)-acetylacetonate, there is employed that excess of acetylacetonate which is necessary for the preparation of the cobalt(III)-acetylacetonate.

4. Process according to one of claims 1 to 3, characterised in that the reaction of the cobalt(II)-compound with acetylacetone is carried out under reflux at the boiling temperature of the solvent.

5. Process according to claim 4, characterised in that the refluxing solvent is conducted over an absorption means for water.

**Revendications**

1. Procédé de préparation d'acétylacétonate de cobalt-(III) par oxydation d'un acétylacétonate de cobalt-(II) dans un solvant organique par du peroxyde d'hydrogène en présence d'acétylacétone, caractérisé en ce qu'on effectue l'oxydation dans une gamme de température comprise entre 40 et 100 °C et en ce qu'on utilise comme solvant organique un solvant organique, miscible à l'eau et inerte à l'égard du peroxyde d'hydrogène à des températures allant jusqu'à 120 °C, solvant dans lequel l'acétylacétonate de cobalt (II) a été préparé et se trouve en solution.

2. Procédé de préparation d'acétylacétonate de cobalt-(III) selon la revendication 1, caractérisé en ce qu'on utilise l'acétone comme solvant.

3. Procédé de préparation d'acétylacétonate de cobalt-(III) selon l'une des revendications 1 ou 2, caractérisé en ce que, lors de la préparation de l'acétylacétonate de cobalt-(II), on travaille avec un excès d'acétylacétonate nécessaire pour la préparation de l'acétylacétonate de cobalt-(III).

4. Procédé selon l'une quelconque des revendications 1 à 3, caractérisé en ce qu'on effectue la réaction du composé de cobalt-(II) avec l'acétylacétone à la température d'ébullition du solvant au reflux.

5. Procédé selon la revendication 4, caractérisé en ce qu'on envoie le solvant qui reflue à travers un agent d'absorption pour l'eau.